(19) Europäisches Patentamt
European Patent Office
Office européen des brevets

(11) Numéro de publication : **0 181 793**
**B1**

# (12) FASCICULE DE BREVET EUROPÉEN

(45) Date de publication du fascicule du brevet : **27.07.88**

(51) Int. Cl.[4] : **C 07 D401/06**, A 61 K 31/415, A 61 K 31/505, A 61 K 31/445

(21) Numéro de dépôt : **85401949.4**

(22) Date de dépôt : **07.10.85**

## (54) Dérivés de pipéridine, leur préparation et leur application en thérapeutique.

(30) Priorité : **16.10.84 FR 8415847**
**10.07.85 FR 8510533**
**10.07.85 FR 8510534**
**10.07.85 FR 8510535**

(43) Date de publication de la demande :
**21.05.86 Bulletin 86/21**

(45) Mention de la délivrance du brevet :
**27.07.88 Bulletin 88/30**

(84) Etats contractants désignés :
**AT BE CH DE FR GB IT LI LU NL SE**

(56) Documents cités :
**EP-A- 0 037 713**
**EP-A- 0 092 391**
**EP-A- 0 098 499**
**Le dossier contient des informations techniques présentées postérieurement au dépôt de la demande et ne figurant pas dans le présent fascicule.**

(73) Titulaire : **SYNTHELABO**
**58, rue de la Glacière**
**F-75621 Paris Cedex 13 (FR)**

(72) Inventeur : **Manoury, Philippe**
**L'Orée de Verrières 38, avenue des Vaupépins**
**F-91370 - Verrieres Le Buisson (FR)**
Inventeur : **Binet, Jean**
**12, Hameau de la Gondole**
**F-91650 - Breuillet (FR)**
Inventeur : **Dewitte, Elisabeth**
**38, avenue d'Orgeval**
**F-95210 - St Gratien (FR)**

(74) Mandataire : **Thouret-Lemaitre, Elisabeth et al**
**Service Brevets - SYNTHELABO 58, rue de la Glacière**
**F-75621 Paris Cédex 13 (FR)**

EP 0 181 793 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention a pour objet des dérivés de pipéridine, leur préparation et leur application en thérapeutique.

Les composés de l'invention répondent à la formule (I) donnée en annexe dans laquelle

$Ar_1$ et $Ar_2$ représentent chacun, indépendamment l'un de l'autre, un radical phényle pouvant porter un atome d'halogène, un radical thiényle ou un radical pyridinyle,

A représente un radical $(C_{2-6})$ alkylène,

X représente un groupe CO ou une liaison,

$R_1$ représente un atome d'hydrogène ou un radical $(C_{1-4})$ alkyle,

soit $R_2$ représente un atome d'hydrogène, et $R_3$ représente un atome d'hydrogène ou un radical hydroxy,

soit $R_2$ et $R_3$ représentent ensemble une liaison,

$R_4$ représente un atome d'hydrogène ou d'halogène.

Les sels d'addition que les composés (I) peuvent former avec les acides pharmaceutiquement acceptables font partie de l'invention.

Les composés préférés de l'invention sont ceux dans lesquels $Ar_1$ et $Ar_2$ représentent chacun, indépendamment l'un de l'autre, un groupe thiényle-2, pyridinyle-2, phényle ou fluoro-4 phényle,

$R_4$ représente un atome d'hydrogène, et les autres radicaux A, X, $R_1$, $R_2$ et $R_3$ ont les significations ci-dessus.

Selon l'invention on peut préparer les composés (I) selon le schéma réactionnel donné en annexe.

On fait réagir un composé (II) avec un composé (III), dans lequel Y est un atome d'halogène ou un groupe partant tel que tosyle ou mésyle, dans un solvant protique tel qu'un alcool ou aprotique tel que la méthylisobutylcétone, à une température de 25 à 120 °C.

Certains des composés (II) sont décrits dans le brevet américain 2 804 222 et dans la littérature par Duncan et al., J. Med. Chem. 13, 1, 1970.

Les composés (II) dans lesquels $Ar_1$ ou $Ar_2$ représente un radical pyridinyle ou phényle peuvent être préparés selon le schéma réactionnel 2 donné en annexe 2 : on fait réagir un composé (IV) avec un composé organométallique (V) comportant le radical $Ar_2$ puis on hydrolyse ou on hydrogénolyse le composé obtenu (VI) pour arriver au composé (II).

Les composés (III) sont décrits dans la littérature, par Vernin et al., J. Het. Chem., 18, 85 (1981).

Les composés (I) dans lesquels $R_2 = R_3 = H$ peuvent être également obtenus par hydrogénolyse des composés (I) dans lesquels $R_2 = H$ et $R_3 = OH$ ou hydrogénation des composés (I) dans lesquels $R_2$ et $R_3$ représentent ensemble une liaison.

Les composés (I) dans lesquels X est CO, $R_1 = H$ et A est $(CH_2)_2$ peuvent être préparés selon le schéma réactionnel 3 donné en annexe 3 : on fait réagir un composé (VII) avec un composé (II) dans un solvant tel que le toluène ou la méthylisobutylcétone.

Les composés (VII) sont décrits dans la littérature.

Les exemples suivants illustrent l'invention.

Les analyses et les spectres IR et RMN confirment la structure des composés.

### Exemple 1 [[(hydroxydiphénylméthyl)-4 pipéridinyl-1]-2 éthyl]-1dihydro-1,3 2H-benzimidazolone-2

On porte à la température du reflux, pendant 18 h, un mélange de 2,7 g (0,01 mole) de α,α-diphénylpipéridine -4-méthanol, 2,4 g (0,01 mole) de (bromo-1 éthyl-2)-1 benzimidazolone-2, 1,2 g de carbonate de sodium ($Na_2CO_3$), et un cristal d'iodure de sodium dans 75 ml de méthylisobutylcétone.

On refroidit le mélange puis évapore à sec. On reprend le résidu d'évaporation avec un mélange d'eau et de chloroforme, on sèche la phase organique, filtre, évapore. On obtient le composé (I) que l'on fait recristalliser dans un mélange (85/15) acétate d'éthyle/ether isopropylique. F = 193-196 °C.

### Exemple 2 [[(diphénylmèthylène)-4 pipéridinyl-1]-2 éthyl]-1dihydro-1,3 2H-benzimidazolone-2

On chauffe à la température du reflux de la méthyl-isobutylcétone, 1,0 g (0,004 mole) de (diphénylméthylène)-4 pipéridine, 0,96 g (0,004 mole) de (bromo-1 éthyl-2)-1 benzimidazolone-2, 0,53 g (0,008 mole) de $Na_2CO_3$ et quelques cristaux de NaI, pendant 9 h.

On évapore le solvant, reprend par de l'eau et du chlorure de méthylène, décante le mélange réactionnel, sèche et évapore la phase organique. On obtient un solide jaune que l'on fait passer sur une colonne de silice (éluant $CH_2Cl_2$ 97/MeOH 3). On évapore et recueille un solide que l'on fait recristalliser dans de l'acétate d'éthyle.

F = 202-203 °C.

### Exemple 3 [[diphénylméthyl-4 pipéridinyl-1]-2 éthyl]-1dihydro-1,3 2H-benzimidazolone-2

On hydrogène à l'aide de l'appareil de Parr, à température ambiante, sous une pression d'hydrogène

de 50psi, en présence de palladium sur carbone à 5 %, une solution de 4 g de [[diphényl méthylène-4 pipéridinyl-1]-2 éthyl]-1 dihydro-1,3 2H-benzimidazolone-2 dans 150 $cm^3$ de méthanol. Lorsque l'absorption d'hydrogène est terminée, on filtre le catalyseur et évapore le filtrat.

On obtient le produit que l'on fait recristalliser dans de l'acétate d'éthyle.

F = 195-196 °C.

Exemple 4 [[[bis(fluoro-4 phényl)méthyl]-4 pipéridinyl-1]-2 éthyl]-1dihydro-1,3 2H-benzimidazolone-2

On chauffe à la température du reflux de la méthyl-isobutyl-cétone, 1,6 g (0,006 mole) de (bromo-1 éthyl-2)-1 dihydro-1,3 2H-benzimidazolone-2, 1,9 g (0,0066 mole) de bis (fluoro-4 phényl)méthyl-4 pipéridine, 0,8 g (0,0075 mole) de $Na_2CO_3$ et quelques cristaux de NaI, pendant environ 6 h. On évapore le solvant, reprend par un mélange d'éther et d'eau, décante le milieu réactionnel, filtre et évapore la phase éthérée. On obtient une huile que l'on fait cristalliser dans de l'éther isopropylique. On filtre un produit blanc pur que l'on sèche.

F = 104-106 °C.

Exemple 5 [[[bis-(fluoro-4 phényl) hydroxy méthyl]-4 pipéridinyl-1]-3propyl]-1 dihydro-1,3 2H-benzimidazolone-2

On chauffe à la température du reflux, dans 60 ml de méthylisobutylcétone 2,1 g (0,007 mole) de [bis(fluoro-4 phényl)-hydroxyméthyl]-4 pipéridine, 1,8 g (0,007 mole) de (bromo-1 propyl-3)-1 dihydro-1,3 2H-benzimidazolone-2, 0,85 g (0,008 mole) de $Na_2CO_3$ et quelques cristaux de NaI pendant 5 h. On évapore le solvant, reprend par un mélange d'eau et de $CH_2Cl_2$, sèche la phase organique et l'évapore. On obtient un résidu solide que l'on chromatographie sur une colonne de silice (éluant : $CH_2Cl_2$/MeOH 97/3, 96/4, puis 94/6).

On évapore les fractions pures et reprend le résidu d'évaporation par de l'éther, filtre un produit blanc que l'on fait cristalliser dans 90 ml d'acétate d'éthyle.

F = 141-143 °C.

Exemple 6 [[[(pyridinyl-2)(phényl)hydroxyméthyl]-4 pipéridinyl-1]-2éthyl]-1 dihydro-1,3 2H-benzimidazolone-2

1.1. [(pyridinyl-2)(phényl)hydroxyméthyl]-4 pipéridine.

1.1.1. [(pyridinyl-2)(phényl)hydroxyméthyl]-4 benzyl-1 pipéridine.

A une solution refroidie à -65 °C de 15,6 ml ($2,5.10^{-2}$ mole) de n-BuLi et de 20 ml tétrahydrofuranne (THF) on ajoute, goutte à goutte, 2,1 ml ($2,2.10^{-2}$ mole) de bromo-2 pyridine en solution dans 10 ml de THF. On agite le mélange 1/4 h à -65 °C puis ajoute, goutte à goutte, 5,6 g ($2.10^{-2}$ mole) de benzyl-1 benzoyl-4 pipéridine dans 20 ml de THF, en maintenant la température à -65 °C. On laisse le mélange se réchauffer très lentement, puis au repos une nuit. On chauffe à la température du reflux 2 h. On évapore à sec, reprend par un mélange d'eau et d'éther. On lave la phase éthérée avec de l'eau, sèche, filtre, évapore.

On chromatographie sur silice (éluant $CH_2Cl_2$/MeOH 95/5). On recueille un produit qui cristallise dans de l'éther de pétrole.

F = 142-143 °C.

1.1.2. [(pyridinyl-2)(phényl)hydroxy-méthyl]-4 pipéridine.

On hydrogène, dans un appareil de Parr, à 40 °C, en présence de palladium sur charbon, sous une pression de 0,35 MPa, 5,4 g ($1,5\ 10^{-2}$ mole) du dérivé obtenu précédemment en solution dans 50 ml de méthanol contenant 1 ml d'acide acétique.

Lorsque l'absorption est terminée, on filtre le catalyseur puis on évapore le mélange à sec.

On reprend le résidu avec un peu d'eau, alcalinise avec de la soude, extrait avec de l'éther. On sèche, filtre, évapore.

On triture le produit qui cristallise dans de l'éther isopropylique.

F = 125-128 °C.

1.2. [[[(pyridinyl-2)(phényl)hydroxyméthyl]-4 pipéridinyl-1]-2 éthyl]-1 dihydro-1,3 2H-benzimidazolone-2.

On introduit dans un ballon 2,5 g ($10^{-2}$ mole) de [(pyridinyl-2)(phényl) hydroxy-méthyl]-4 pipéridine, 2,5 g ($10^{-2}$ mole) de (bromo-1 éthyl-2)-1 dihydro-1,3 2H-benzimidazolone-2, 1,2 g de $Na_2CO_3$, 1 cristal de NaI et 75 ml de méthylisobutylcétone.

On maintient le mélange à la température de reflux pendant 4 h et on évapore à sec. On reprend le résidu avec un mélange d'eau et de chlorure de méthylène ; on sèche la phase organique, filtre et

évapore.

Par trituration dans de l'éther, on obtient un produit que l'on fait recristalliser dans de la méthyléthylcétone.

F = 206-207 °C.

Exemple 7 [[[diphényl-hydroxyméthyl]-4 pipéridinyl-1]-2éthyl]-3 1H, 3H-quinazolinedione-2,4

Dans un ballon tricol de 100 ml, on introduit 2 g (0,00748 mole) de (diphénylhydroxyméthyl)-4 pipéridine et 1,4 g (0,00748 mole) de dihydro-2,3 5H-oxazolo (2,5-b) quinazolinone-5 dans 50 ml de toluène contenant une goutte d'acide chlorhydrique 7,5 N. On chauffe le mélange à 100-120 °C pendant 28 h.

On filtre le précipité formé, le lave avec un peu de toluène et le sèche. On fait recristalliser le composé dans du méthanol.

F = 206-208 °C.

Les composés préparés à titre d'exemples sont représentés dans le tableau suivant.

(voir tableaux p.5 et suiv.)

Tableau

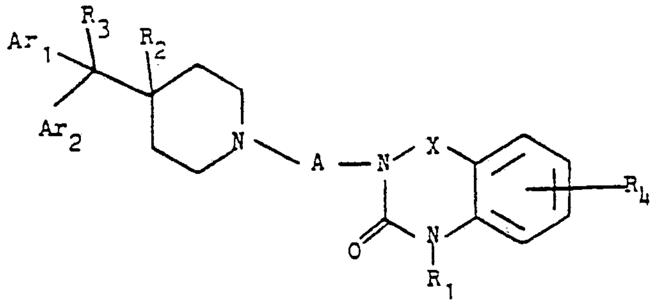

| Composé | X | A | $Ar_1$ | $Ar_2$ | $R_1$ | $R_2$ | $R_3$ | $R_4$ | F(°C) |
|---|---|---|---|---|---|---|---|---|---|
| 1 | liaison | $(CH_2)_2$ | $C_6H_5$ | $C_6H_5$ | H | H | H | H | 195-196 |
| 2 | liaison | $(CH_2)_2$ | $C_6H_5$ | $C_6H_5$ | H | liaison | | H | 202-203 |
| 3 | liaison | $(CH_2)_2$ | $C_6H_5$ | $C_6H_5$ | H | H | OH | H | 193-196 |
| 4 | liaison | $(CH_2)_2$ | $C_6H_5$ | $C_6H_5$ | $CH_3$ | H | OH | H | 166-170 |
| 5 | liaison | $(CH_2)_2$ | $C_6H_5$ | $C_6H_5$ | iPr | H | OH | H | 140-142 |
| 6 | liaison | $(CH_2)_3$ | $C_6H_5$ | $C_6H_5$ | H | H | OH | H | 180-182 |
| 7 | liaison | $(CH_2)_4$ | $C_6H_5$ | $C_6H_5$ | H | H | OH | H | 95 |
| 8 | liaison | $(CH_2)_5$ | $C_6H_5$ | $C_6H_5$ | H | H | OH | H | 114-117 |
| 9 | liaison | $(CH_2)_2$ | 4-F-$C_6H_4$ | 4-F-$C_6H_4$ | H | H | H | H | 104-106 |
| 10 | liaison | $(CH_2)_2$ | 4-F-$C_6H_4$ | 4-F-$C_6H_4$ | H | H | OH | H | 149-151 |
| 11 | liaison | $(CH_2)_3$ | 4-F-$C_6H_4$ | 4-F-$C_6H_4$ | H | H | OH | H | 141-143 |
| 12 | liaison | $(CH_2)_4$ | 4-F-$C_6H_4$ | 4-F-$C_6H_4$ | H | H | OH | H | 110 |
| 13 | liaison | $(CH_2)_3$ | 4-F-$C_6H_4$ | $C_6H_5$ | H | H | OH | H | 138-140 |
| 14 | liaison | $(CH_2)_3$ | 4-F-$C_6H_4$ | 4-F-$C_6H_4$ | H | H | H | H | 165-167 |
| 15 | liaison | $(CH_2)_3$ | 4-F-$C_6H_4$ | 4-F-$C_6H_4$ | H | liaison | | H | 171-172 |
| 16** | liaison | $(CH_2)_4$ | 4-F-$C_6H_4$ | 4-F-$C_6H_4$ | H | H | H | H | 210-212 |
| 17 | liaison | $(CH_2)_2$ | $C_6H_5$ | Pyrid-2 | H | H | OH | H | 206-207 |
| 18 | liaison | $(CH_2)_3$ | $C_6H_5$ | Pyrid-2 * | H | H | OH | H | 201-203 |
| 19 | liaison | $(CH_2)_3$ | 4-F-$C_6H_4$ | Pyrid-2 | H | H | OH | H | 192-195 |
| 20 | liaison | $(CH_2)_2$ | Thiényl-2 | Thiényl-2 | H | H | OH | H | 209-211 |
| 21 | liaison | $(CH_2)_3$ | Thiényl-2 | Thiényl-2 | H | H | OH | H | 224-226 |

Tableau (Suite)

| Composé | X | A | $Ar_1$ | $Ar_2$ | $R_1$ | $R_2$ | $R_3$ | $R_4$ | F(°C) |
|---|---|---|---|---|---|---|---|---|---|
| 22 | liaison | $(CH_2)_4$ | $4\text{-}F\text{-}C_6H_4$ | Pyrid-2 | H | H | OH | H | 69 |
| 23 | CO | $(CH_2)_2$ | $C_6H_5$ | $C_6H_5$ | H | H | OH | H | 206-208 |
| 24 | CO | $(CH_2)_3$ | $C_6H_5$ | $C_6H_5$ | H | H | OH | H | 221-223 |
| 25 | CO | $(CH_2)_4$ | $C_6H_5$ | $C_6H_5$ | H | H | OH | H | 207-208 |
| 26 | CO | $(CH_2)_2$ | $4\text{-}F\text{-}C_6H_4$ | $4\text{-}F\text{-}C_6H_4$ | H | H | OH | H | 213-214 |
| 27 | CO | $(CH_2)_3$ | $4\text{-}F\text{-}C_6H_4$ | $4\text{-}F\text{-}C_6H_4$ | H | H | OH | H | 209-212 |
| 28 | liaison | $(CH_2)_2$ | $C_6H_5$ | $C_6H_5$ | $CH_3$ | H | OH | Cl | 122-125 |

* Pyrid = Pyridinyl
** point de fusion de l'oxalate

Les composés ont été soumis à divers essais pharmacologiques montrant leur activité antagoniste de l'histamine et de la sérotonine.

1. Activité in vitro : iléon isolé de cobaye.

Le test a été effectué selon la méthode de Magnus modifiée par Savini (Arch. Int. Pharmacodyn., 113, 157), sur des cobayes tricolores mâles pesant environ 300 g, à jeun depuis 18 heures.

Un fragment d'iléon est prélevé, placé à 39 °C dans un bain de tyrode traversé par un courant de carbogène ($O_2$ 95 %, $CO_2$ 5 %) et relié à un capteur isotonique avec une tension maximale de 2,5 g. Les contractions sont enregistrées à l'aide d'un microdynamomètre Ugo Basile.

Les contractions sont induites par les divers agents spasmogènes dont la concentration provoquant une réponse submaximale est déterminée (histamine : 1 à $8.10^{-8}$ g/ml). Les composés de l'invention dissous dans de l'eau distillée ou une solution 0,1 N d'acide méthanesulfonique sont mis en contact avec l'iléon pendant 1 mn avant l'introduction de la substance spasmogène.

Les $CA_{50}$ (concentration diminuant de 50 % les contractions induites par l'histamine) de certains composés de l'invention vont de $10^{-7}$ à $10^{-8}$ molaire.

2. Activité in vivo : inflammation induite par l'histamine ou la sérotonine.

L'injection intraplantaire dans une des pattes postérieures du rat d'histamine (200 µg) ou de sérotonine (1 µg), provoque un œdème mesuré, 1 h après l'injection, à l'aide d'un pléthysmomètre à mercure Ugo Basile.

Les composés de l'invention, mis en suspension dans du tween en solution à 1 % dans de l'eau distillée sont administrés p.o. (0,5 ml/100 g) 1 h avant l'injection de l'agent inflammatoire.

Les $DA_{40}$ (dose qui diminue de 40 % le volume de l'œdème) sont mesurées.

Les composés de l'invention ont une $DA_{40}$ allant de 1 à 5 mg/kg lorsque l'agent inflammatoire est l'histamine. Certains composés de l'invention sont actifs à une dose $DA_{40}$ allant de 0,2 à 1 mg/kg lorsque l'agent inflammatoire est la sérotonine.

Les composés de l'invention peuvent donc être utilisés comme antiallergiques, antiprurigineux pour le traitement des allergies respiratoires telles que rhinites, rhumes des foins, allergies cutanées telles que dermatites, urticaires, allergies oculaires, œdème de Quincke et manifestations allergiques diverses.

Les dérivés de l'invention plus spécifiquement actifs comme antagonistes de la sérotonine peuvent être utilisés pour lutter contre certains effets indésirables de ce médiateur au niveau périphérique ou au niveau central. Ils sont destinés en particulier au traitement de la migraine.

L'invention comprend, par conséquent, toutes compositions pharmaceutiques renfermant les composés et/ou leurs sels comme principes actifs, en association avec tous excipients appropriés à leur administration, en particulier par voie orale ou parentérale.

Les voies d'administration peuvent être les voies orale et parentérale.

La posologie quotidienne peut aller de 5 à 200 mg.

Annexe 1

Schéma réactionnel 1

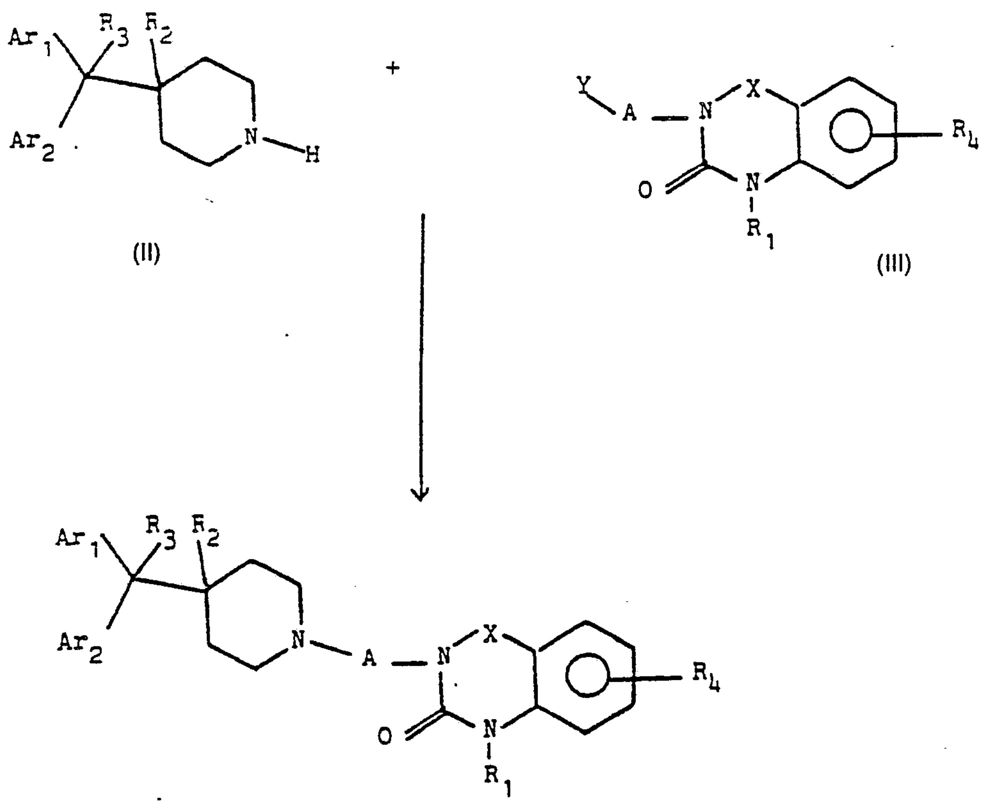

(II) (III) (I)

Annexe 2

Schéma réactionnel 2

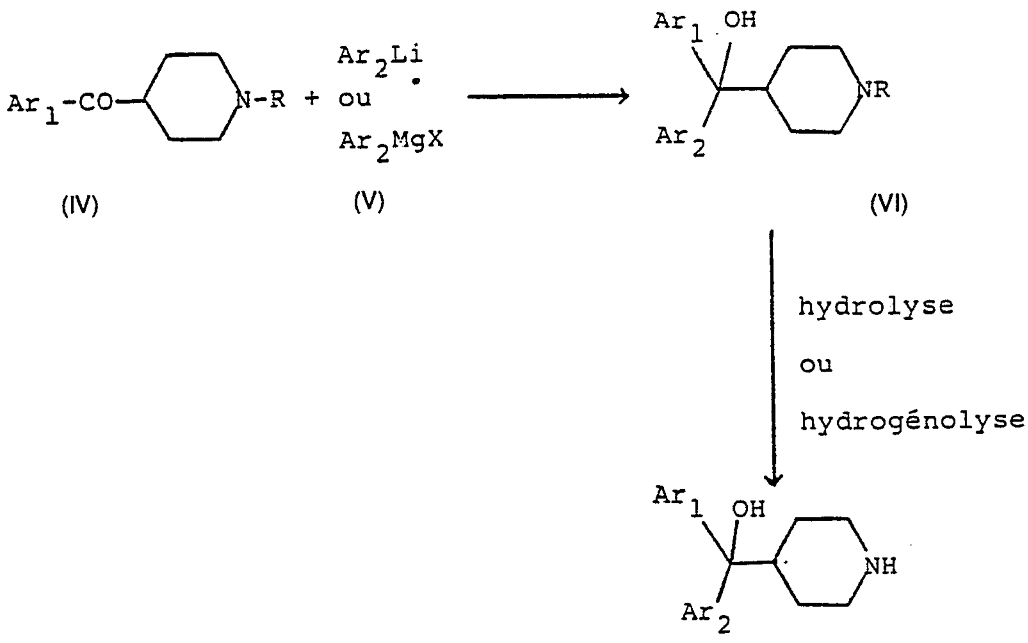


(IV) (V) (VI) (II)

R = groupe protecteur tel que acétyle ou benzyle

Annexe 3

Schéma réactionnel 3

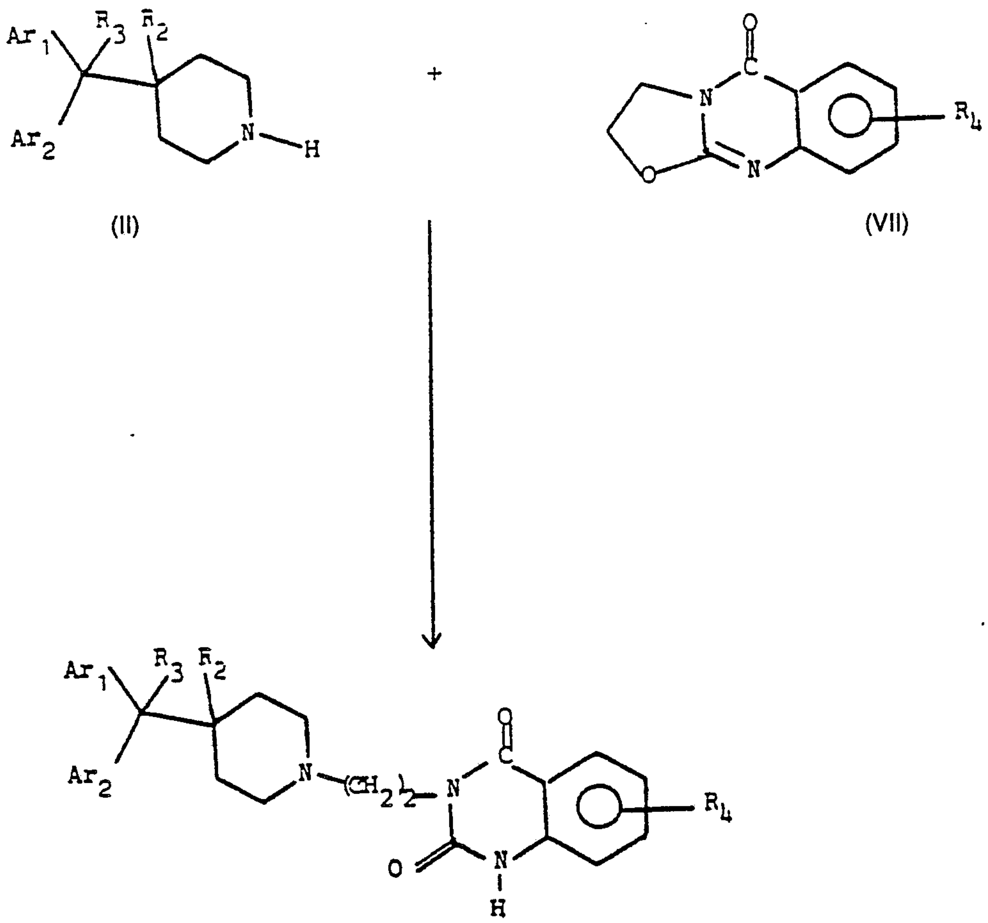

(II)

(VII)

(I, $R_1$=H)

**Revendications** (pour les Etats contractants : BE, CH, DE, FR, GB, IT, LI, LU, NL, SE)

1. Dérivés de pipéridine répondant à la formule (I)

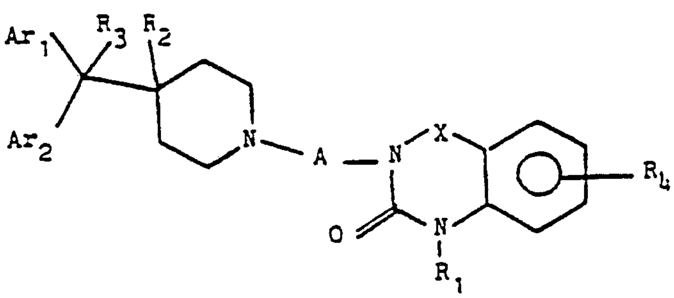

(I)

dans laquelle

$Ar_1$ et $Ar_2$ représentent chacun, indépendamment l'un de l'autre, un radical phényle pouvant porter un atome d'halogène, un radical thiényle ou un radical pyridinyle,

A représente un radical ($C_{2-6}$) alkylène,

X représente un groupe CO ou une liaison

$R_1$ représente un atome d'hydrogène ou un radical ($C_{1-4}$) alkyle,

soit $R_2$ représente un atome d'hydrogène et $R_3$ représente un atome d'hydrogène ou un radical hydroxy,

soit $R_2$ et $R_3$ représentent ensemble une liaison,

$R_4$ représente un atome d'hydrogène ou d'halogène,

ainsi que les sels d'addition que peuvent former les composés (I) avec les acides pharmaceutiques acceptables.

2. Dérivés selon la revendication 1, dans lesquels

$Ar_1$ et $Ar_2$ représentent chacun, indépendamment l'un de l'autre, un groupe thiényle-2, pyridinyle-2, phényle ou fluoro-4 phényle,

$R_4$ représente un atome d'hydrogène,

et les autres radicaux A, X, $R_1$, $R_2$ et $R_3$ ont les significations données dans la revendication 1.

3. Dérivés selon la revendication 1, dans lesquels

$Ar_1$ et $Ar_2$ représentent chacun, indépendamment l'un de l'autre, un groupe thiényle-2, pyridinyle-2, phényle ou fluoro-4 phényle,

$R_4$ représente un atome d'hydrogène,

$R_2$ est H et $R_3$ est OH.

4. Dérivés selon la revendication 2 ou 3, dans lesquels $Ar_1$ représente un groupe fluoro-4 phényle et $Ar_2$ un autre groupe fluoro-4 phényle ou pyridinyle-2.

5. Procédé de préparation des composés selon la revendication 1, procédé caractérisé en ce que l'on fait réagir un composé de formule (II)

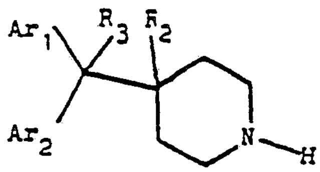

(II)

avec un composé de formule (III)

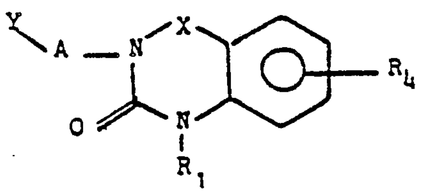

(III)

dans lequel Y est un atome d'halogène ou un groupe partant, les autres radicaux ayant les significations données dans la revendication 1, dans un solvant protique ou aprotique, à une température de 25 à 120 °C.

6. Composition pharmaceutique caractérisée en ce qu'elle contient un composé tel que spécifié dans l'une quelconque des revendications 1 à 4 en association avec tout excipient approprié.

**Revendication** (pour l'Etat contractant AT)

Procédé de préparation de dérivés de pipéridine répondant à la formule (I)

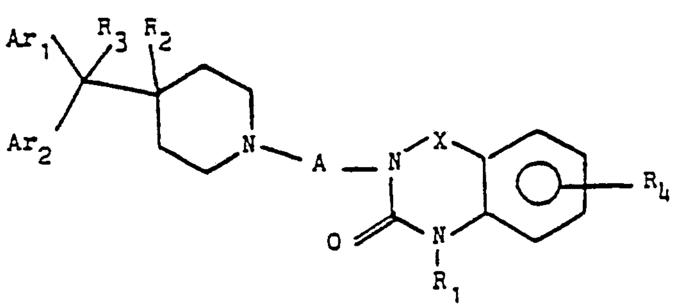

(I)

dans laquelle

$Ar_1$ et $Ar_2$ représentent chacun, indépendamment l'un de l'autre, un radical phényle pouvant porter un atome d'halogène, un radical thiényle ou un radical pyridinyle,

A représente un radical ($C_{2-6}$) alkylène,

X représente un groupe CO ou une liaison,

$R_1$ représente un atome d'hydrogène ou un radical $(C_{1-4})$ alkyle,

soit $R_2$ représente un atome d'hydrogène et $R_3$ représente un atome d'hydrogène ou un radical hydroxy,

soit $R_2$ et $R_3$ représentent ensemble une liaison,

$R_4$ représente un atome d'hydrogène ou d'halogène,

ainsi que des sels d'addition que peuvent former les composés (I) avec les acides pharmaceutiquement acceptables, procédé caractérisé en ce que l'on fait réagir un composé de formule (II)

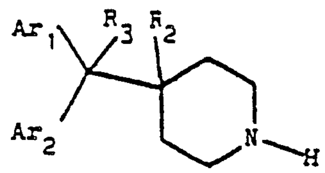

(II)

avec un composé de formule (III)

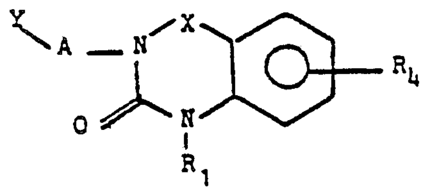

(III)

dans lequel Y est un atome d'halogène ou un groupe partant, les autres radicaux ayant les significations données ci-dessus, dans un solvant protique ou aprotique à une température de 25 à 120 °C.

**Claims** (for the Contracting States : BE, CH, DE, FR, GB, IT, LI, LU, NL, SE)

1. Piperidine derivatives corresponding to the formula (I)

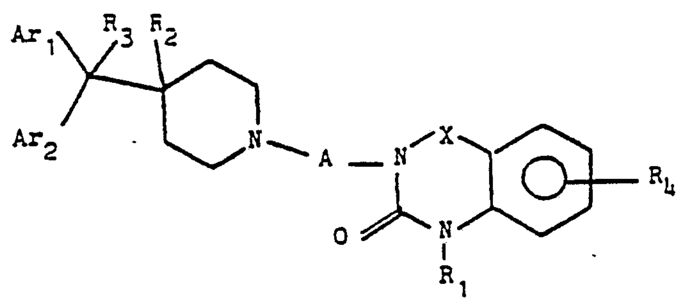

(I)

in which

$Ar_1$ and $Ar_2$ each denote, independently of each other, a phenyl radical capable of bearing a halogen atom, a thienyl radical or a pyridinyl radical,

A denotes a $(C_{2-6})$ alkylene radical,

X denotes a CO group or a bond,

$R_1$ denotes a hydrogen atom or a $(C_{1-4})$ alkyl radical,

either $R_2$ denotes a hydrogen atom and $R_3$ denotes a hydrogen atom or a hydroxyl radical,

or $R_2$ and $R_3$ together denote a bond, and

$R_4$ denotes a hydrogen or halogen atom,

and the addition salts which the compounds (I) can form with pharmaceutically acceptable acids.

2. Derivatives according to Claim 1, in which

$Ar_1$ and $Ar_2$ each denote, independently of each other, a 2-thienyl, 2-pyridinyl, phenyl or 4 fluorophenyl group,

$R_4$ denotes a hydrogen atom

and the other radicals A, X, $R_1$, $R_2$ and $R_3$ have the meanings given in Claim 1.

3. Derivatives according to Claim 1, in which $Ar_1$ and $Ar_2$ each denote, independently of each other, a 2-thienyl, 2-pyridinyl, phenyl or 4-fluorophenyl group, $R_4$ denotes a hydrogen atom, $R_2$ is H and $R_3$ is OH.

4. Derivatives according to Claim 2 or 3, in which $Ar_1$ denotes a 4-fluorophenyl group and $Ar_2$ another 4-fluorophenyl or a 2-pyridinyl group.

5. Process for preparing the compounds according to Claim 1, which process is characterized in that a compound of formula (II)

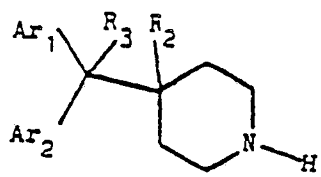

(II)

is reacted with a compound of formula (III)

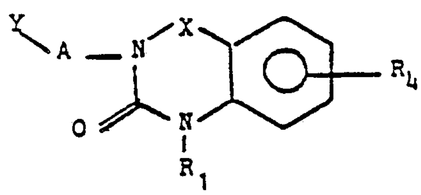

(III)

in which Y is a halogen atom or a labile group, the other radicals having the meanings given in Claim 1, in a protic or aprotic solvent, at a temperature of 25 to 120 °C.

6. Pharmaceutical composition characterized in that it contains a compound such as specified in any one of Claims 1 to 4, in combination with any suitable excipient.

**Claim** (for the Contracting State AT)

Process for preparing piperidine derivatives corresponding to the formula (I)

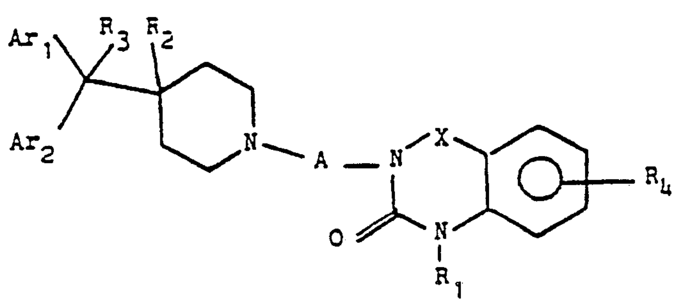

(I)

in which

$Ar_1$ and $Ar_2$ each denote, independently of each other, a phenyl radical capable of bearing a halogen atom, a thienyl radical or a pyridinyl radical,

A denotes a $(C_{2-6})$ alkylene radical,

X denotes a CO group or a bond,

$R_1$ denotes a hydrogen atom or a $(C_{1-4})$ alkyl radical,

either $R_2$ denotes a hydrogen atom and $R_3$ denotes a hydrogen atom or a hydroxyl radical,

or $R_2$ and $R_3$ together denote a bond, and

$R_4$ denotes a hydrogen or halogen atom,

and the addition salts which the compounds (I) can form with pharmaceutically acceptable acids, which process is characterized in that a compound of formula (II)

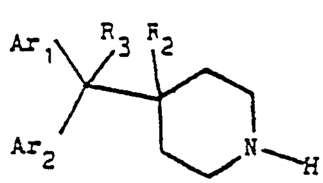

(II)

is reacted with a compound of formula (III)

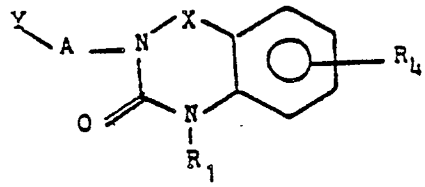

(III)

in which Y is a halogen atom or a labile group, the other radicals having the meanings given above, in a protic or aprotic solvent, at a temperature of 25 to 120 °C.

**Patentansprüche** (für die Vertragsstaaten : BE, CH, DE, FR, GB, IT, LI, LU, NL, SE)

1. Piperidinderivate gemäß der Formel (I)

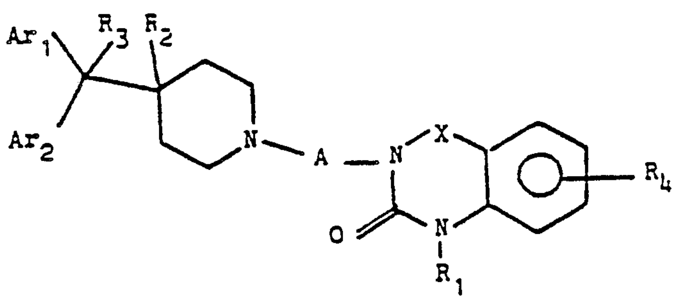

(I)

in welcher
$Ar_1$ und $Ar_2$ jeweils unabhängig voneinander für einen Phenylrest, der ein Halogenatom tragen kann, einen Thienylrest oder einen Pyridinylrest stehen,
A einen $(C_{2-6})$-Alkylenrest,
X eine CO-Gruppe oder eine Bindung und
$R_1$ ein Wasserstoffatom oder einen $(C_{1-4})$-Alkylrest bedeuten,
$R_2$ entweder für ein Wasserstoffatom und $R_3$ für ein Wasserstoffatom oder einen Hydroxyrest steht oder
$R_2$ und $R_3$ gemeinsam eine Bindung bedeuten, und
$R_4$ für ein Wasserstoffatom oder ein Halogenatom steht,
sowie die Additionssalze, die die Verbindungen (I) mit den pharmazeutisch verwendbaren Säuren bilden können.

2. Derivate nach Anpruch 1, in welchen
$Ar_1$ und $Ar_2$ jeweils unabhängig voneinander eine 2-Thienyl-, 2-Pyridinyl-, Phenyl- oder 4-Fluorophenylgruppe bedeuten,
$R_4$ für ein Wasserstoffatom steht
und die anderen Reste A, X, $R_1$, $R_2$ und $R_3$ die in Anspruch 1 angegebene Bedeutung haben.

3. Derivate nach Anspruch 1, in welchen $Ar_1$ und $Ar_2$ jeweils unabhändig voneinander für eine 2-Thienyl-, 2-Pyridinyl-, Phenyl- oder 4-Fluorophenylgruppe stehen, $R_4$ ein Wasserstoffatom bedeutet, $R_2$ für H und $R_3$ für OH steht.

4. Derivate nach Anspruch 2 oder 3, in welchen $Ar_1$ für eine 4-Fluorophenylgruppe und $Ar_2$ für eine weitere 4-Fluorophenylgruppe oder eine 2-Pyridinylgruppe steht.

5. Verfahren zur Herstellung von Verbindungen nach Anspruch 1, dadurch gekennzeichnet, daß man eine Verbindung der Formel II

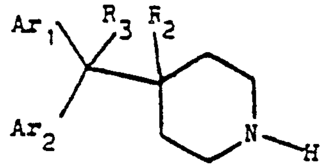

(II)

mit einer Verbindung der Formel (III)

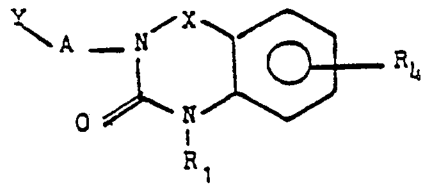

(III)

in welcher Y für ein Halogenatom oder eine Abgangsgruppe steht und die anderen Reste die in Anspruch 1 angegebene Bedeutung haben, in einem protischen oder aprotischen Lösungsmittel bei einer Temperatur von 25 bis 120 °C reagieren läßt.

6. Pharmazeutische Zusammensetzung, dadurch gekennzeichnet, daß sie eine Verbindung nach irgendeinem der Ansprüche 1 bis 4 in Kombination mit jedem geeigneten Bindemittel enthält.

**Patentanspruch** (für den Vertragsstaat AT)

Verfahren zur Herstellung von Piperidinderivaten gemäß der Formel (I)

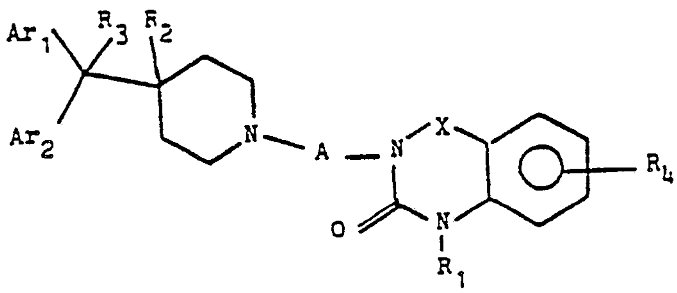

(I)

in welcher

$Ar_1$ und $Ar_2$ jeweils unabhängig voneinander für einen Phenylrest, der ein Halogenatom tragen kann, einen Thienylrest oder einen Pyridinylrest stehen,

A einen $(C_{2-6})$-Alkylenrest,

X eine CO-Gruppe oder eine Bindung und

$R_1$ ein Wasserstoffatom oder einen $(C_{1-4})$-Alkylrest bedeuten,

$R_2$ entweder für ein Wasserstoffatom und $R_3$ für ein Wasserstoffatom oder einen Hydroxyrest steht oder

$R_2$ und $R_3$ gemeinsam eine Bindung bedeuten, und

$R_4$ für ein Wasserstoffatom oder ein Halogenatom steht,

sowie der Additionssalze, die die Verbindungen (I) mit den pharmazeutisch verwendbaren Säuren bilden können, dadurch gekennzeichnet, daß man eine Verbindung der Formel II

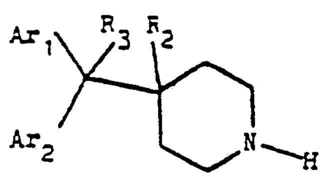

(II)

mit einer Verbindung der Formel (III)

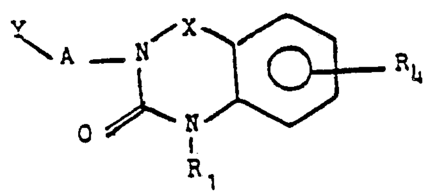

(III)

in welcher Y für ein Halogenatom oder eine Abgangsgruppe steht und die anderen Reste die oben angegebene Bedeutung haben, in einem protischen oder aprotischen Lösungsmittel bei einer Temperatur von 25 bis 120 °C reagieren läßt.